# EUROPÄISCHE PATENTSCHRIFT

(11) **EP 2 132 294 B1**
(45) Veröffentlichungstag und Bekanntmachung des Hinweises auf die Patenterteilung: **26.09.2012**
(21) Anmeldenummer: 07847763.5
(22) Anmeldetag: 04.12.2007
(51) Int. Cl.: C11D 3/50, C11D 17/00, A61K 8/04, A61K 8/11, A61Q 13/00

(54) **WIRKSTOFFTRÄGERSYSTEME**
ACTIVE INGREDIENT CARRIER SYSTEMS
SYSTÈMES VECTEURS DE SUBSTANCES ACTIVES

(30) Priorität: 28.02.2007 DE 102007010109
(43) Veröffentlichungstag der Anmeldung: 16.12.2009
(73) Patentinhaber: Henkel AG & Co. KGaA, 40589 Düsseldorf (DE)
(72) Erfinder: ARTIGA GONZALEZ, Rene-Andres, 40589 Düsseldorf (DE); BAUER, Andreas, 41564 Kaarst (DE); HILSMANN, Jürgen, 40589 Düsseldorf (DE); STURM, Mario, 51371 Leverkusen (DE); DREJA, Michael, 41469 Neuss (Norf) (DE)
(86) Internationale Anmeldenummer: PCT/EP2007/063259
(87) Internationale Veröffentlichungsnummer: WO 2008/104240

(56) Entgegenhaltungen:
- EP-A- 1 195 429
- WO-A-02/062937
- WO-A-2007/014601
- US-A1- 2007 031 502
- US-B1- 6 649 414

## Beschreibung

Die vorliegende Erfindung betrifft einen partikelförmiger Träger, welcher zumindest teilweise mit feinpartikulären Wirkstoffträgermatrices belegt ist und ein Verfahren zu seiner Herstellung bzw. zur Immobilisierung feinpartikulärer Wirkstoffträgermatrices, wie vorzugsweise Mikrokapseln, auf anorganischem Trägermaterial.

Die Erfindung betrifft ferner eine Detergenszusammensetzung, enthaltend derartige Träger. Außerdem betrifft die Erfindung ein Verfahren zum Aufbringen von feinpartikulären Wirkstoffträgermatrices auf Textilien sowie ein Verfahren zum Beduften von Textilien, welches sich der feinpartikulären Wirkstoffträgermatrices bzw. der partikelförmigen Träger bedient.

Wirkstoffträgersysteme in feinpartikulärer Form, wie z.B. Mikrokapseln, sowie deren Einsatz in Kosmetika, aber auch in Wasch- und Reinigungsmitteln, sind an sich bekannt. Diese Systeme sollen vor allem dazu dienen, bestimmte Wirkstoffe, wie beispielsweise Parfüm, zum einen vor Umgebungseinflüssen zu bewahren und zum anderen den oder die Wirkstoffe gezielt erst am Anwendungsort freizusetzen.

Insbesondere im Zusammenhang mit dem Einsatz von entsprechenden feinpartikulären Wirkstoffträgersystemen in festen Wasch- oder Reinigungsmitteln sind bestimmte Probleme vorhanden. Viele dieser feinpartikulären Wirkstoffträgersysteme, insbesondere aber die klassischen Mikro- oder Nanokapseln, können nur in wäßrigen Systemen, insbesondere Suspensionen, hergestellt, gelagert und auch in den Handel gebracht werden. Daher müssen solchen System z.B. stets Konservierungsmittel zugegeben werden, was aber oft nicht erwünscht ist. Außerdem bereitet auch die eigentliche Einarbeitung der Wirkstoffträgersysteme in feste Wasch- oder Reinigungsmitteln als solche erhebliche Probleme. Erstens können die in Form wäßriger Systeme vorliegenden feinpartikulären Wirkstoffträgersysteme nicht ohne weiteres in eine feste Waschmittelmatrix, welche u.a. wasserempfindliche Rohstoffe wie Percarbonat oder andere hydratbildende Salze enthalten, eingearbeitet werden, ohne eine gewisse Dekompensierung des Waschmittels zu bewirken. Zweitens ist das Unterbringen der wäßrigen Systeme nur unter hohem Energieeinsatz möglich, um ein Klumpen und eine schlechte Rieselfähigkeit des resultierenden Gemisches zu vermeiden. Dies führt aber zur Zerstörung der feinpartikulären Wirkstoffträgersysteme, so dass diese ihrem eigentlichen Zwecke nicht mehr dienen können, nämlich dem Schutz von Wirkstoffen und deren gezielte Freisetzung am Anwendungsort.

Daher bestand Bedarf an einer Bereitstellungsform feinpartikulärer Wirkstoffträgersysteme, welche leicht und stabil in feste Wasch- oder Reinigungsmittel eingearbeitet werden können. Die Aufgabe der Erfindung war es, dieses Bedürfnis zu befriedigen.

Diese Aufgabe wurde in überraschender Weise durch die Bereitstellung eines partikelförmigen Trägers, welcher zumindest teilweise mit mikrokapseln, enthaltend Riech stoffe, belegt ist, gelöst.

Mit diesem Gegenstand geht als weiterer Vorteil einher, dass die auf dem partikelförmigen Träger immobilisierten feinpartikulären Wirkstoffträgermatrices auch während der Lagerung und bei der Verwendung im Endprodukt, z.B. einer vollwertigen Waschmittelformulierung, stabilisiert werden. Dadurch wird verhindert, dass der Gehalt an Wirkstoffen bei der Lagerung gemindert wird und dass sich die Wirkstoffe bei der Lagerung zersetzen, also beispielsweise oxidieren, oder unangenehme Eigengerüche freisetzen. Außerdem verfügen die auf dem partikelförmigen Träger immobilisierten feinpartikulären Wirkstoffträgermatrices über günstige Freigabecharakteristika für die enthaltenen Wirkstoffe.

Bei den feinpartikulären Wirkstoffträgermatrices kann es sich um alle möglichen Mikroträger bzw. Mikroformkörper handeln, welche einen Riechstoff (Parfüm), enthalten, wobei die Größe (mittlerer Durchmesser) der feinpartikulären Wirkstoffträgermatrices im Bereich von vorzugsweise 0,01 µm bis 2000 µm, vorteilhafterweise 0,1 µm bis 1500 µm, in vorteilhafterer Weise, 1 µm bis 1000 µm, in noch vorteilhafterer Weise 10 µm bis 800 µm, in weiter vorteilhafter Weise 100 µm bis 700 µm liegt. Mögliche Untergrenzen können beispielsweise auch bei 200 µm oder auch bei 300 µm liegen. Mögliche Obergrenzen können beispielsweise auch bei 600 µm, 500 µm, 400 µm, 300 µm, 200 µm, 100 µm, 50 µm oder auch bei nur 20 µm liegen. Besonders geeignet kann z.B. auch eine Größe im Bereich von 100-400 µm sein oder auch eine Größe im Bereich kleiner 100 µm, beispielsweise 1µm bis 80 µm, 1µm bis 60 µm, 1µm bis 40 µm oder auch 1µm bis 20 µm. Die feinpartikulären Wirkstoffträgermatrices haben vorzugsweise eine überwiegend sphärische Form.

Nach einer bevorzugten Ausführungsform der Erfindung handelt es sich bei den Wirkstoffträgermatrices um Partikel, bei welchen eine Trennung in Kern und Hüllbereich vorliegt, wobei es sich bei den Wirkstoffträgermatrices um Mikro- und/oder Nanokapseln handelt, insbesondere um Mikrokapseln mit Partikeldurchmessern im zuvor angegebenen Bereich. Die Kapseln als solche sowie deren Herstellung sind dem Fachmann seit langem bekannt und brauchen daher an dieser Stelle nicht in besonders breiter Form erläutert werden.

Unter dem Begriff "Mikrokapsel" werden vorzugsweise solche Aggregate verstanden, die mindestens einen festen, halbfesten oder flüssigen Kern enthalten, der von mindestens einer kontinuierlichen Hülle, insbesondere einer Hülle aus Polymer(en), umschlossen ist. Üblicherweise handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Neben einkernigen Mikrokapseln sind auch mehrkernige Aggregate bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Bevorzugt sind einkernige Mikrokapseln mit einer kontinuierlichen Hülle. Die Hülle kann beispielsweise aus natürlichen, halbsynthetischen oder synthetischen Materialien bestehen. Natürlich Hüllmaterialien sind beispielsweise Gummi arabicum, Agar Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Im Inneren der Mikrokapseln können beliebige Stoffe, vorzugsweise empfindliche, chemisch oder physikalisch inkompatible sowie flüchtige Komponenten (bzw. Wirkstoffe), insbesondere aus dem Bereich der Wasch- und Reinigungsmittelinhaltsstoffe, lager- und transportstabil eingeschlossen werden. In den Mikrokapseln können beispielsweise optische Aufheller, Tenside, Komplexbildner, Bleichmittel, Bleichaktivatoren, Farb- und Duftstoffe, Antioxidantien, Gerüststoffe, Enzyme, Enzym-Stabilisatoren, antimikrobielle Wirkstoffe, Vergrauungsinhibitoren, Antiredepositionsmittel, pH-Stellmittel, Elektrolyte, Schauminhibitoren, Vitamine, Proteine enthalten sein. Die Füllungen der Mikrokapseln können z.B. Feststoffe oder Flüssigkeiten z.B. in Form von Lösungen oder Emulsionen bzw. Suspensionen sein.

Die Mikrokapseln können im herstellungsbedingten Rahmen eine beliebige Form aufweisen, sie sind jedoch bevorzugt näherungsweise kugelförmig. Ihr Durchmesser entlang ihrer größten räumlichen Ausdehnung kann je nach den in ihrem Inneren enthaltenen Komponenten und der Anwendung im bereits weiter oben genannten Bereich liegen, z.B. zwischen 0,01 µm (visuell nicht als Kapsel erkennbar) und 2000 µm liegen. Bevorzugt können beispielsweise auch sichtbare Mikrokapseln mit einem Durchmesser im Bereich von z.B. 100 µm bis 1.000 µm, insbesondere von 400 µm bis 900 µm sein. Die Mikrokapseln sind nach allen bekannten Verfahren zugänglich, wobei der Koazervation und der Grenzflächenpolymerisation die größte Bedeutung zukommt.

Nach einer weiteren bevorzugten Ausführungsform der Erfindung handelt es sich bei den Wirkstoffträgermatrices um Partikel, bei welchen keine Trennung in Kern und Hüllbereich vorliegt, wobei es sich bei den Wirkstoffträgermatrices vorzugsweise um Mikrosphärulen handelt, insbesondere mit Partikeldurchmessern im bereits zuvor genannten Bereich. Mikrosphärulen sind den Mikrokapseln verwandte Systeme, bei denen aber keine exakte Trennung in Kern- und Hüllbereich vorliegt. In Mikrosphärulen liegt der eingebettete Wirkstoff in fester, d. h. dispergierter, oder gelöster Form in die Trägermatrix inkorporiert vor. Mikrosphärulen sind also eine Sonderform von Mikrokapseln.

Dabei ist es auch möglich, vorzugsweise wenn der Wirkstoff in fester, dispergierter Form vorliegt, dass mehrere Phasen in den Mikrosphärulen beobachtet werden können.

Vorteilhafterweise können auch verschiedene Wirkstoffträgermatrices zusammen eingesetzt werden, z.B. Mikrokapseln gemeinsam mit Mikrosphärulen.

Erfindungsgemäß bevorzugt einsetzbare Mikrosphärulen sind z.B. Zusammensetzungen aus
(a) einem oder mehreren Fettalkoholen, vorzugsweise in Mengen von 5-90 Gew.-%, insbesondere 40-80 Gew.-%, vorteilhafterweise gewählt aus der Gruppe der verzweigten oder unverzweigten Alkylalkohole mit 16-22 C-Atomen, beispielsweise Cetylalkohol, Stearylalkohol, Cetyl-/Stearylalkohol und Behenylalkohol,
(b) einem oder mehreren Wachsestern, vorzugsweise in Mengen von 1-90 Gew.-%, insbesondere 2-50 Gew.-%, vorzugsweise Ester höhermolekularer wasserunlöslicher Fettalkohole und Fettsäuren, insbesondere solche Wachsester, bei denen die Anzahl der C-Atome größer ist als etwa 22, und höhermolekularer Fettsäuren, bei welchen die Anzahl der C-Atome größer ist als etwa 14, vorteilhafterweise Cetylpalmitat
(c) gegebenenfalls hochdispersem Siliciumdioxid, vorzugsweise in Mengen von 0-20 Gew.-%, insbesondere 0,5-5 Gew.-%, bevorzugt gewählt aus der Gruppe der hochreinen, röntgenamorphen Siliciumdioxidsorten, insbesondere solchen, die durch Hydrolyse von SiCl₄ in einer Knallgasflamme erzeugt werden können, besonders bevorzugt aus der Gruppe der Produkte, welche die Handelsbezeichnung Aerosil® tragen,
(d) sowie Riechstoffe (Parfümöle)

Die Mikrosphärulen können auf einfache Weise nach an sich bekannten Verfahren hergestellt werden. Als besonders günstiges Herstellungsverfahren hat sich die sogenannte Sprühtrocknung bzw. Sprüherstarrung erwiesen. Es können die für solche Verfahren üblichen und gängigen Apparaturen verwendet werden.

Beispielsweise können die Mikrosphärulen dadurch erhalten werden, dass eine Schmelze aus
(a) einem oder mehreren Fettalkoholen, gewählt aus der Gruppe der verzweigten oder unverzweigten Alkylalkohole mit 16-22 C-Atomen,
(b) einem oder mehreren Wachsestem,
(c) gegebenenfalls hochdispersem Siliciumdioxid,
(d) gegebenenfalls einen oder mehrere Wirkstoffe, welche in gelöster oder dispergierter Form vorliegen, enthaltend,
(e) sowie gegebenenfalls übliche pharmazeutische bzw. kosmetische Hilfs- und/oder Zusatzstoffen enthaltend,
mechanisch zerteilt wird und die zerteilte Schmelze zum Erstarren gebracht wird.

Die mechanische Zerteilung der Schmelze kann z.B. dadurch erfolgen, dass die Schmelze mittels einer Düse, einer Zerstäuberscheibe oder einen Zweistoffdüsenzerstäuber zu feinen Tröpfchen zerstäubt wird. Durch möglichst rasches Abkühlen bis zum Erstarren werden aus den so erzeugten Tröpfchen die endgültigen Mikrosphärulen erzeugt. Es ist günstig, die Abkühlung so zu gestalten, dass die Schmelze bzw. Dispersionsschmelze in ein Kühlgas, beispielsweise Luft oder ein Inertgas, versprüht wird. Die Ableitung des mikropartikulären Sprühproduktes erfolgt vorteilhaft mit dem Kühlgasstrom, der durch einen nachgeschalteten Exhaustor aufrechterhalten werden kann.

In einer bevorzugten Ausführungsform der Erfindung enthalten die feinpartikulären Wirkstoffträgermatrices, vorzugsweise Mikrokapseln, feste, halbfeste oder flüssige Stoffe.

Nach einer weiteren bevorzugten Ausführungsform der Erfindung zeichnet sich der erfindungsgemäße partikelförmige Träger dadurch aus , dass er anorganisches Trägermaterial umfasst, vorzugsweise ausgewählt aus der Gruppe Zeolithe, Sulfate, Carbonate, Silikate, Tone, Kieselsäure und/oder deren Gemische, wobei es, insbesondere mit Blick auf Anwendungen im Wasch- und Reinigungsmittelbereich vorteilhaft ist, wenn das Trägermaterial sprühgetrocknetes Material umfasst, was ebenfalls einer bevorzugten Ausführungsform der Erfindung entspricht.

Ein besonders bevorzugter Träger enthält z.B. Zeolith, insbesondere Zeolith A, sowie Carbonat, insbesondere Soda, sowie Sulfat, insbesondere Natriumsulfat, vorteilhafterweise in Mengen von je zumindest 5 Gew.-%, vorzugsweise von je zumindest 10 Gew.-%, Gew.-% jeweils bezogen auf den Träger ohne die feinpartikulären Wirkstoffträgermatrices. Sulfat, insbesondere Natriumsulfat, ist vorteilhafterweise auch in größeren Mengen in dem Träger enthalten, z.B. in Mengen > 15 Gew.-%, > 20 Gew.-%, 25 Gew.-%, 30 Gew.-%, 35 Gew.-% oder sogar > 40 Gew.-%, Gew.-% bezogen auf den Träger ohne die feinpartikulären Wirkstoffträgermatrices. Eine mögliche Obergrenze für Sulfat liegt z.B. bei 50 Gew.-%.

Auch Carbonat, insbesondere Soda, ist vorteilhafterweise in größeren Mengen in dem Träger enthalten, z.B. in Mengen > 15 Gew.-%, > 20 Gew.-%, 25 Gew.-% oder 30 Gew.-%, Gew.-% bezogen auf den Träger ohne die feinpartikulären Wirkstoffträgermatrices. Eine mögliche Obergrenze für Carbonat liegt z.B. bei 40 Gew.-%.

Ein besonders bevorzugter Träger kann auch Polycarboxylate enthalten, z.B. Homopolymerisate der Acrylsäure sowie Copolymerisate der Acrylsäure und Maleinsäure, insbesondere in Mengen > 0,1 Gew.-%, >1 Gew.-%, >2 Gew.-%, >3 Gew.-%, >4 Gew.-% oder >5 Gew.-%, Gew.-% bezogen auf den Träger ohne die feinpartikulären Wirkstoffträgermatrices. Eine Obergrenze für das Polycarboxylat kann z.B. bei 20 Gew.-% oder 10 Gew.-% oder 5 Gew.-% liegen

Ein besonders bevorzugter Träger kann auch Niotenside enthalten, z.B. ethoxylierte Fettalkohole, beispielsweise mit 12 bis 18 Kohlenstoffatomen und einem Ethoxylierungsgrad im Bereich von 5-25 , insbesondere in Mengen > 0,01 Gew.-%, >0,1 Gew.-%, >0,2 Gew.-%, >0,3 Gew.-%, >0,4 Gew.-% oder >0,5 Gew.-%, Gew.-% bezogen auf den Träger ohne die feinpartikulären Wirkstoffträgermatrices. Eine Obergrenze für das Niotenside kann z.B. bei 5 Gew.-% oder 2 Gew.-% oder 1 Gew.-% liegen.

Ein besonders bevorzugter Träger kann auch Celluloseether enthalten, z.B. Carboxymethylcellulose, insbesondere in Mengen > 0, 1 Gew.-%, >0,5 Gew.-%, >1 Gew.-%, >1,5 Gew.-% oder >2 Gew.-%, Gew.-% bezogen auf den Träger ohne die feinpartikulären Wirkstoffträgermatrices. Eine Obergrenze für den Celluloseether kann z.B. bei 5 Gew.-% oder 4 Gew.-% oder 3 Gew.-% liegen

Ein besonders bevorzugter Träger kann folglich zumindest einer der folgenden Komponenten beinhalten, aber vorzugsweise alle Komponenten "a-f" oder zumindest 5 dieser Komponenten, z.B. "a-d, f" oder "a-c, e, f" oder "a, b, d-f", insbesondere aber zumindest 2 der Komponenten aus "c, e, f", beispielsweise zumindest "c, e" oder "c, f" oder "e, f", vorteilhafterweise aber zumindest die Komponenten "c, e, f":
(a) Polycarboxylate, z.B. Homopolymerisate der Acrylsäure sowie Copolymerisate der Acrylsäure und Maleinsäure, vorzugsweise in einem der zuvor genannten Mengenbereiche,
(b) Niotenside, z.B. ethoxylierte Fettalkohole, vorzugsweise in einem der zuvor genannten Mengenbereiche,
(c) Zeolith, z.B. Zeolith A, vorzugsweise in Mengen > 1 Gew.-%, >3 Gew.-%, >5 Gew.-%, >10 Gew.-% oder > 15 Gew.-%, Gew.- % bezogen auf den Träger ohne die feinpartikulären Wirkstoffträgermatrices, bei einer Obergrenze von beispielsweise 50 Gew.-% oder 40 Gew.-% oder 30 Gew.-% oder 20 Gew.-%,
(d) Celluloseether, z.B. Carboxymethylcellulose, vorzugsweise in einem der zuvor genannten Mengenbereiche.
(e) Carbonate, z.B. Soda, vorzugsweise in einem der zuvor genannten Mengenbereiche,
(f) Sulfate, Z.B. Natriumsulfat, vorzugsweise in einem der zuvor genannten Mengenbereiche.

Das sprühgetrocknete Material kann man z.B. erhalten, wenn ein zu trocknendes Material (flüssige Lösung oder Suspension, z.B. eine wässrige Aufschlämmung thermisch stabiler Waschmittelinhaltsstoffe, die sich unter den Bedingungen der Sprühtrocknung weder verflüchtigen noch zersetzen wie z.B. Tenside, Gerüststoffe, Stellmittel) am oberen Ende eines weiten, zylindrischen Behälters durch Düsen oder mittels einer schnell rotierenden Zerstäuberscheibe zu einem feinen Nebel versprüht. Dem entstehenden Sprühkegel wird z.B. heiße Luft (z.B. mit einer Temperatur von 250 bis 350°C) oder auch ein Inertgas von unten entgegengeführt. Die Zufuhr des Trocknungsgases kann auch im Gleichstrom von oben erfolgen, z.B. bei sehr temperaturempfindlichen Produkten (z.B. Enzyme, aktive Mikroorganismen). Das Trockengut fällt als mehr oder weniger feines Pulver, als Granulat oder in Form kleiner Perlen (Prills) nach unten und wird am Boden des Trockners ausgetragen.

Wenn der erfindungsgemäße partikelförmige Träger anorganisches Trägermaterial in einer Gesamtmenge von mindestens 40 Gew.-%, vorzugsweise von mindestens 50 Gew.-%, vorteilhafterweise von mindestens 60 Gew.-%, insbesondere von mindestens 70 Gew.-% in dem Träger umfasst, Gew.-% bezogen auf den gesamten Träger, so liegt wiederum eine bevorzugte Ausführungsform der Erfindung vor. Eine mögliche Obergrenze für das enthaltene anorganische Trägermaterial liegt z.B. bei 80 Gew.-%.

Wenn der erfindungsgemäße partikelförmige Träger eine Partikelgröße im Bereich von 0,05 bis 2,5 mm aufweist, vorzugsweise von 0,1 bis 2,0 mm, insbesondere von 0,2 bis 1,6 mm, so liegt ebenfalls eine bevorzugte Ausführungsform der Erfindung vor.

Nach einer weiteren bevorzugten Ausführungsform der Erfindung ist der erfindungsgemäße partikelförmige Träger als solcher mit einer Flüssigkeit imprägniert, vorzugsweise umfassend
i. Riechstoffe (Parfümöle)
ii. flüssige Wasch- und Reinigungsmittelinhaltsstoffe, wie vorzugsweise Tenside, insbesondere Niotenside, Silikonöle, Paraffine
iii. flüssige Kosmetikinhaltstoffe, wie vorzugsweise Öle
iv. flüssige nicht-pharmazeutische Additive oder Wirkstoffe und/oder
Mischungen vorgenannter,
wobei die Flüssigkeit insbesondere zwischen 0,1 und 30 Gew.-% des Gesamtgewichtes des Trägers ausmacht, beispielsweise 1- 20 Gew.-%, 3-15 Gew.-% oder auch 5-10 Gew.-%.

Als Riechstoffe bzw. Parfümöle können im Rahmen der Erfindung beispielsweise einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyciohexylacetat, Linalylacetat, Dimethylbenzylcarbinyl-acetat, Phenylethylacetat, Linalylbenzoat, Benzylformiat, Ethylmethylphenylglycinat, Allylcyciohexylpropionat, Styrallylpropionat und Benzyisalicylat. Zu den Ethern zählen beispielsweise Benzylethylether, zu den Aldehyden z.B. die linearen Alkanale mit 8 - 18 C-Atomen, Citral, Citronellal, Citronellyloxyacetaldehyd, Cyclamenaldehyd, Hydroxycitronellel, Lilial und Bourgeonal, zu den Ketonen z.B. die Jonone, Isomethylionon und Methyl-cedrylketon, zu den Alkoholen Anethol, Citronellol, Eugenol, Geraniol, Linalool, Phenylethylelkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Die einsetzbaren Parfümöle können selbstverständlich auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Galbanumöl und Ladanumöl.

Wie schon beschrieben wurde, ist der erfindungsgemäße partikelförmige Träger z.B. mit Mikrokapseln als feinpartikuläre Wirkstoffträgermatrices belegt. Wenn dabei das Kapselmaterial der Mikrokapseln ausgewählt ist aus
(a) Homopolymeren, vorzugsweise
   - durch Polymerisation von Vinyl-Gruppen-haltigen Monomeren zugängliche Polymere, wie z.B. Polyvinylacetate, Polyvinylalkohol, Polyvinylpyrrolidon,
   - Polycarboxylate, Polycarbonsäuren, z.B. Polyacrylsäure, Polymethacrylsäure,
   - Polysulfonsäuren, z.B. Polystyrolsulfonsäure,
   - Polyester, z.b. Polyacrylate, Glycolpolyacrylat,
   - Polyamide, z.B. Polyacrylamide,
   - Polyurethane,
   - Polyethylenoxide, Polypropylenoxide oder ander Polyalkylenglycolderivative;
(b) Polykondensate, vorzugsweise
   - ethoxylierte Phenol-Formaldehyd-Harze
   - sulfonierte aromatische Formaldehyd-Harze
   - Harnstoff oder Melamin- Formaldehyd-Harze, z.B. Melamin-Harnstoff-Formaldehyd-Harze, Melamin-Phenol-Formaldehyd-Harze
   - Polyamid-Harze, Polyamin-Harze, und Epoxid-Harze
(c) AB-Copolymere vorzugsweise
   - Styrolcopolymere, z.B. Styrol-Acrylsäure-Polymere oderr Styrol-Ethylenoxid-Polymere
   - Copolymer von Polyvinyl- und Maleinsäure-Compounds, z.B. Styrol-Maleinsäureanhydrid-Polymer
   - Polyvinyl-Polyalkylen-Ccopolymere, z.B. Vinylacetat
   - Ethylenpolymer, Ethylen-Acrylsäure-Acrylsäureester-Polymere oder Ethylen-Acrylsäure-Acrylonitril-Polymere
   - andere Vinylcopolymere, z.B. Vinylacetatpolymere, Acrylsäure-Acrylonitril-Polymere, Acrylsäure-Acrylamid-Polymere;
(d) ABA-Blockcopolymer, wobei vorzugsweise
   - "A" für wasserlösliche oder wasserquellbare Gruppen wie Polyethylenoxid, Polyvinylalkohol, Polyacrylamid, Polyacrylsäure, Poly vinylpyrrolidon oder Polyccaprolacton,
   - "B" für weniger oder kaum wasserlösliche Gruppen so wie Polypropylenoxid, Polyvinylacetat, Polyvinylbutyral, Polylaurylmethacrylat, Polystyrol, Polyhydroxystearinsäure, Polysiloxan steht
(e) B(A)ₙ Pfropf(co)polymere, wobei vorzugsweise
   - "A" für wasserlösliche oder wasserquellbare Gruppen wie Vinylalkohol, Vinylacetate, Ethylenoxide, Propylenoxid, Vinylsulphonate, Acrylsäuren und Vinylamine, und
   - "B" für Vinylpolymer-Ketten oder Siloxan-Ketten steht,
(f) natürliche oder abgewandelte natürliche Polymere z.B.Cellulosederivative, wie Carboxymethylcellulose, Hydroxypropylmethylcellulose,
Methylcellulose, Ethylcellulose, Celluloseacetat, Cellulosenitrat, Eiweißverbindungen (Gelatine, Albumin, Casein),
so liegt eine bevorzugte Ausführungsform der Erfindung vor.

Die erfindungsgemäß einsetzbaren Mikrokapseln sind vorzugsweise durch Einkapselung flüssiger, halbfester oder fester Phasen durch Umhüllung mit filmbildenden Polymeren, die sich nach Emulgierung und Koazervation oder Grenzflächenpolymerisation auf dem einzuhüllenden Material niederschlagen, erhältlich. Der Einsatz solcher Mikrokapseln entspricht einer bevorzugten Ausführungsform der Erfindung.

Wenn die erfindungsgemäß einzusetzenden Mikrokapseln, bei thermischer, mechanischer, chemischer oder enzymatischer Einwirkung ihren Inhalt freisetzen, insbesondere bei Bedingungen wie sie beim Trocknen in einem automatischen Wäschetrockner oder beim Bügeln vorliegen, so liegt eine bevorzugte Ausführungsform der Erfindung vor. Die mechanische Einwirkung, die zum Freisetzen des Inhalts Mikrokapseln, führt, kann z.B. ein manuelles Reiben oder Rubbeln an dem Textil sein.

Bevorzugt einsetzbare Riechstoffe (Parfümöle) wurden schon weiter oben genannt.

Bevorzugte flüssige Kosmetikinhaltstoffe sind z.B. Öle, vorteilhafterweise vollsynthetische Öle wie z.B. Siliconöle, pflanzliche und/oder tierische fette Öle (Triglyceride mittlerer oder ungesättigter Fettsäuren) und/oder etherische Öle (z.B. aus Pflanzenteilen).

Die feinpartikulären Wirkstoffträgermatrices, vorzugsweise Mikrokapseln, insbesondere die darin enthaltene Flüssigkeit, können vorzugsweise ein oder mehrere hautpflegende und/oder hautschützende Aktivstoffe enthalten. Hautpflegende Aktivstoffe sind alle solchen Aktivstoffe die der Haut einen sensorischen und/oder kosmetischen Vorteil verleihen. Hautpflegende Aktivstoffe sind bevorzugt ausgewählt aus den nachfolgenden Substanzen:
a) Wachse wie beispielsweise Carnauba, Spermaceti, Bienenwachs, Lanolin und/oder Derivate derselben und andere.
b) Hydrophobe Pflanzenextrakte
c) Kohlenwasserstoffe wie beispielsweise Squalene und/oder Squalane
d) Höhere Fettsäuren, vorzugsweise solche mit wenigstens 12 Kohlenstoffatomen, beispielsweise Laurinsäure, Stearinsäure, Behensäure, Myristinsäure, Palmitinsäure, Ölsäure, Linolsäure, Linolensäure, Isostearinsäure und/oder mehrfach ungesättigte Fettsäuren und andere.
e) Höhere Fettalkohole, vorzugsweise solche mit wenigstens 12 Kohlenstoffatomen, beispielsweise Laurylalkohol, Cetylalkohol, Stearylalkohol, Oleylalkohol, Behenylalkohol, Cholesterol und/oder 2-Hexadecanaol und andere.
f) Ester, vorzugsweise solche wie Cetyloctanoate, Lauryllactate, Myristyllactate, Cetyllactate, Isopropylmyristate, Myristylmyristate, Isopropylpalmitate, Isopropyladipate, Butylstearate, Decyloleate, Cholesterolisostearate, Glycerolmonostearate, Glyceroldistearate, Glyceroltristearate, Alkyllactate, Alkylcitrate und/oder Alkyltartrate und andere.
g) Lipide wie beispielsweise Cholesterol, Ceramide und/oder Saccharoseester und andere.
h) Vitamine wie beispielsweise die Vitamine A, C und E, Vitaminalkylester, einschließlich Vitamin-C-Alkylester und andere.
i) Sonnenschutzmittel
j) Phospholipide
k) Derivate von alpha-Hydroxysäuren
l) Riechstoffe
m) Germizide für den kosmetischen Gebrauch, sowohl synthetische wie beispielsweise Salicylsäure und/oder andere als auch natürliche wie beispielsweise Neemöl und/oder andere.
n) Silikone
sowie Mischungen jeglicher vorgenannter Komponenten.

Ein erfindungsgemäßer partikelförmiger Träger, welcher vorteilhafterweise weniger als 50 Gew.-%, 40 Gew.-% oder 30 Gew.-%, beispielsweise zwischen 0,1 -15 Gew.-% seines Gewichts an feinpartikulären Wirkstoffträgermatrices, insbesondere Mikrokapseln, enthält, vorzugsweise zwischen 1 -10 Gew.-% , insbesondere zwischen 1,5 -5 Gew.-%, bezogen auf den gesamten Träger inklusive feinpartikulärer Wirkstoffträgermatrices, stellt wiederum eine bevorzugte Ausführungsform der Erfindung dar.

Es ist auch möglich, dass ein erfindungsgemäßer Träger, der mit Mikrokapseln, belegt ist, außerdem noch beschichtet ist, vorzugsweise abgepudert und/oder mit einem Film gecoatet. Dies entspricht einer bevorzugten Ausführungsform der Erfindung. Um eine solche Ausführungsform der Erfindung zu verwirklichen, werden vorzugsweise zuerst feinpartikuläre Wirkstoffträgermatrices auf dem partikelförmigen Träger immobilisiert und der resultierende mit feinpartikulären Wirkstoffträgermatrices beladene partikelförmigen Träger anschließend beschichtet, also vorzugsweise abgepudert und/oder mit einem Film gecoatet.

Die vorzugsweise einsetzbare Beschichtung kann farbige Substanzen, Farbstoffe, Aufheller und/oder Pigmente, vorteilhafterweise im nanoskaligen Bereich oder im Mikrometerbereich, aufweisen, was einer bevorzugten Ausführungsform der Erfindung entspricht.

Im Sinne der zuvor genannten Beschichtung kann es auch vorteilhaft sein, dass der erfindungsgemäße Träger mit einem Thermoplasten, wie vorzugsweise PEG, PVA, Polyacrylate, PVP, Kohlenhydrate, Polyester wie vorzugsweise PET gecoatet ist. Dies entspricht einer weiteren bevorzugten Ausführungsform der Erfindung.

Geeignete optionale Beschichtungsmittel können z.B. wasserlösliche, wasserdispergierbare und/oder wasserunlösliche (Co-)Polymere enthalten. Die optionale Coatingschicht als solche kann wasserlöslich oder wasserunlöslich sein. Nachfolgend werden einige optional einsetzbare Polymere erläutert.

Wasserlösliche Polymere enthalten eine für die Wasserlöslichkeit ausreichende Anzahl an hydrophilen Gruppen und sind vorteilhafterweise nicht vernetzt. Die hydrophilen Gruppen können nichtionisch, anionisch, kationisch oder zwitterionisch sein, z.B. -NH₂, -OH, -SH, -O-, -COOH, -COO⁻M⁺, -SO3⁻M⁺, -PO₃²⁻M²⁺, -NH₃⁺, 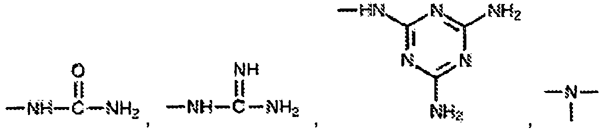 usw.

Die einzelnen Polymere können gleichzeitig unterschiedliche hydrophile Gruppen enthalten, z.B. ionische und nichtionische und/oder anionionische neben kationischen Gruppen.

Bevorzugte wasserlösliche Polymere können z.B. natürliche Polysaccharide und/oder Polypeptide, wie z.B. Stärke, Alginate, Pektine, Pflanzengummen, Caseine, Gelatine usw. sein.

Bevorzugte wasserlösliche Polymere können z.B. halbsynthetische Polymere, wie z.B. Celluloseether oder Stärkeether sein.

Bevorzugte wasserlösliche Polymere können z.B. biotechnologisch erzeugte Produkte, wie z.B. Pullulan, Curdlan oder Xanthan sein.

Bevorzugte wasserlösliche Polymere können z.B. synthetische Polymere, wie z.B. Homo- und/oder Copolymere der (Meth)acrylsäure und ihrer Derivate, der Malein-, Vinylsulfon-, Vinylphosphonsäure, Polyvinylalkohol, Polyethylenimin, Polyvinylpyrrolidon u.a. sein.

Bevorzugte Überzugsmittel enthalten wasserlösliches (Co-)Polymer, insbesondere mit einem Schmelz- oder Erweichungspunkt im Bereich von 48°C bis 300°C, vorteilhafterweise im Bereich von 48°C bis 200°C, in weiter vorteilhafter Weise im Bereich von 48°C bis 200°C.

Geeignetes wasserlösliche (Co-)Polymer mit entsprechendem Schmelz- oder Erweichungspunkt kann vorteilhafterweise aus der Gruppe bestehend aus Polyalkylenglykolen, Polyethylenterephthalaten, Polyvinylalkoholen und Mischungen daraus ausgewählt werden.

Das optionale Coating kann neben dem eigentlichen Überzugsmittel oder auch unabhängig von diesem weitere Bestandteile umfassen, so z.B. vorteilhafterweise Textil-weichmachende Verbindungen und/oder Parfüm.

Nach einer bevorzugten Ausführungsform der Erfindung enthält die optionale Beschichtung des partikelförmigen Trägers Lipide und/oder Silikonöle.

Bevorzugte Lipide sind
(a) lipophile Kohlenwasserstoffe (wie beispielsweise auch Triacontan, Squalen oder Carotinoide usw.),
(b) lipophile Alkohole (wie beispielsweise Wachsalkohole, Retinol oder Cholesterin usw.),
(c) Etherlipide
(d) lipophile Carbonsäuren (Fettsäuren),
(e) lipophile Ester [wie Neutralfette - d.h. Mono-, Di- u. Triacylglycerine (Triglyceride), Sterinester usw.]
(f) lipophile Amide (wie z.B. Ceramide usw.),
(g) Wachse
(h) Lipide mit mehr als 2 Hydrolyseprodukten, wie z.B. Glykolipide, Phospholipide, Sphingolipide und/oder Glycerolipide usw.
(i) Lipide in Form höhermolekularer Konjugate mit mehr als 2 Hydrolyseprodukten, wie z.B. Lipoproteinenund/oder Lipopolysaccharide usw.,
(j) Phosphor-freie Glykolipide, wie z.B. Glykosphingolipide (wie vorzugsweiseCerebroside, Ganglioside, Sulfatide) oder wie z.B. Glykoglycerolipide (wie vorzugsweise Glykosyldi- und - monoglyceride)usw.
(k) Kohlenhydrat-freie Phospholipide, wie z.B. Sphingophospholipide (wie vorzugsweise Sphingomyelinen) oder wie z.B. Glycerophospholipide (wie vorzugsweise Lecithine, Kephaline, Cardiolipine, Phosphatidylinosite und -inositphosphate usw.)
(l) Mischungen aus Vorgenannten.

Vorgenannte Lipide und/oder Silikonöle können auch in dem Träger oder in den feinpartikulären Wirkstoffträgermatrices, vorzugsweise Mikrokapseln, enthalten sein.

Ein erfindungsgemäßer partikelförmiger Träger, welcher zumindest teilweise mit feinpartikulären Wirkstoffträgermatrices belegt ist, kann beispielsweise durch Mischen einer wäßrigen Suspension von feinpartikulären Wirkstoffträgermatrices, vorzugsweise Mikrokapseln, mit einem übertrockneten anorganischen Träger in einem Mischer mit vorzugsweise niedrigem Energieeintrag, wie insbesondere einem Paddelmischer erhalten werden. Dies entspricht einer bevorzugten Ausführungsform der Erfindung.

Selbstverständlich können die eingesetzten wäßrigen Suspensionen auch Hilfsmittel, wie z.B. sogenannte Suspendierhilfen enthalten, z.B. ionische (vorzugsweise anionaktive) grenzflächenaktive Stoffe, die als Dispergiermittel und Antiabsetzmittel wirken, indem sie die Benetzung der suspendierten Partikel mit dem Dispersionsmittel erhöhen.

Das Mischen läuft vorzugsweise bei Froude-Zahlen im Bereich von 0,1 bis 25, vorteilhafterweise von 0,5 bis 15, in weiter vorteilhafter Weise von 1 bis 10, in vorteilhafterer Weise von 1,5 bis 8, insbesondere von 2 bis 4 in gewünschter Weise ab. Die Froude-Zahl ist durch die Beziehung (w² - r)/g gegeben (w = Winkelgeschwindigkeit, r = Länge der Werkzeuge ab Mittelachse, g = Erdbeschleunigung). Das Arbeiten bei diesen Froude-Zahlen entspricht einer bevorzugten Ausführungsform der Erfindung.

Wenn bei der Herstellung des erfindungsgemäßen partikelförmigen Trägers ein übertrockneter anorganischer Träger mit einem Wassergehalt < 10 Gew.-%, vorzugsweise < 8 Gew.-%, vorteilhafterweise < 6 Gew.-%, in noch vorteilhafterweise < 5 Gew.-%, insbesondere < 4 Gew.-% oder < 3 Gew.-%, bezogen auf den eingesetzten übertrockneten anorganischen Träger, eingesetzt wird, so liegt wiederum eine bevorzugte Ausführungsform der Erfindung vor. Insbesondere geeignet können z.B Wassergehalte von 2-3 Gew.-% sein.

Ein anorganischer Träger ist dann übertrocknet, wenn er einen Wassergehalt aufweist, der unter seinem "normalen" Gleichgewichts-Wassergehalt liegt. Dies wird nachfolgend veranschaulicht.

Bevorzugte Trägermaterialien enthalten oder sind z.B. die Zeolithe, insbesondere Zeolith A, Zeolith P, Zeolith MAP und Zeolith X und Zeolith Y . In einer besonders bevorzugten Ausführungsform hat der Zeolith z.B.die Formel: Na₁₂[(AlO₂)₁₂(SiO₂)₁₂]·xH2O worin x von etwa 18 bis etwa 30 reicht. Dieses Material ist als Zeolith A bekannt. In seinem Gleichgewichtszustand enthält Zeolith A rund 22 Gew.-% Hydratwasser. "Übertrocknete" Zeolithe (z.B. x ≤ 17, vorzugsweise x ≤ 16, insbesondere x ≤ 15) weisen also einen Feuchtigkeitsgehalt auf, der geringer als sein Gleichgewichtsgehalt ist. Der Gleichgewichtsgehalt ist der bei Einwirkung von höheren Temperaturen, bei Zeolith z.B. bei 800°C entfernbare Gesamtwassergehalt. Ein bevorzugter, übertrockneter Zeolith weist einen Feuchtigkeitsgehalt (entfembar bei 800°C) auf, der vorzugsweise 15 Gew.-% nicht übersteigt, beispielsweise 7-15 Gew.-5 beträgt, aber auch Werte < 7 Gew.-% annehmen kann, z.b. 5 Gew.-%.

Beispielsweise kann man aus "normalem", also hydratisierten Zeolith A bei 135°C etwa 75 Gew.-% des Wassergehalts entfernen, so dass er bei 135°C z.B. auf einen Feuchtigkeitsgehalt von etwa 7 Gew.-% getrocknet werden kann. Ein solcher Zeolith A ist bevorzugt. Ein anorganischer Träger, der z.B. solchen Zeolith enthält, gilt als übertrocknet im Sinne der Erfindung.

Das Gesagte gilt analog auch für alle anderen Träger(materialien), z.B. für Zeolith MAP. Dieser weist einen "normalen" Gleichgewichts-Wassergehalt von rund 19 Gew.-% auf, übertrockneter Zeolith MAP weist dagegen einen verminderten Wassergehalt auf, z.B. 5-13 Gew.-%.

Beispielsweise können auch übertrocknete amorphe Natriumsilikate mit einem Modul Na20 : SiO2 von 1 : 2 bis 1 : 3,3, vorzugsweise von 1 : 2 bis 1 : 2,8 und insbesondere von 1 : 2 bis 1 : 2,6, die Wassergehalte unter 15 Gew.-%, bezogen auf das Gewicht des Silikats, aufweisen, eingesetzt werden. Herkömmliche amorphe Silikate, sogenannte "Wassergläser" weisen dagegen Restwassergehalte zwischen 17 und 20 Gew.-% auf.

Der Begriff "übertrocknet" macht im Rahmen dieser Erfindung also kenntlich, dass der "normale" Gleichgewichts-Wassergehalt des eingesetzten Trägers oder Trägermaterials unterschritten wird.

Der bei dem Verfahren vorteilhafterweise eingesetzte übertrocknete anorganische Träger oder diesen bildende Stoffe, zeichnen sich gemäß einer bevorzugten Ausführungsform der Erfindung dadurch aus, dass der normale Gleichgewichts-Wassergehalt des Trägers bzw. der Gesamtheit seiner Inhaltsstoffe um zumindest 10%, vorzugsweise um zumindest 20 %, vorteilhafterweise um zumindest 30%, insbesondere um zumindest 40% oder sogar zumindest 50% unterschritten ist.

Wenn bei der Herstellung des erfindungsgemäßen partikelförmigen Trägers die dabei eingesetzte wässrige Suspension von Mikrokapseln, zumindest 30 Gew.-%, vorzugsweise zumindest 40 Gew.-%, insbesondere zumindest 50 Gew.-% Mikrokapseln, enthält, so liegt wiederum eine bevorzugte Ausführungsform der Erfindung vor.

Außerdem ist es bevorzugt, wenn der erfindungsgemäße partikelförmige Träger wenigstens eine üblicherweise in Wasch- oder Reinigungsmitteln enthaltene Substanz, vorzugsweise eine Substanz aus der Gruppe der Tenside, Buildersubstanzen (anorganische und organische Buildersubstanzen), Bleichmittel, Bleichaktivatoren, Bleichstabilisatoren, Bleichkatalysatoren, Enzyme, spezielle Polymere (beispielsweise solche mit Cobuilder-Eigenschaften), Vergrauungsinhibitoren, optische Aufheller, UV-Schutzsubstanzen, Soil Repellents, Elektrolyte, Farbmittel, Riechstoffe, Duftstoffe, Parfümträger, pH-Stellmittel, Komplexbildner, Fluoreszenzmittel, Schauminhibitoren, Knitterschutzmittel, Antioxidantien, quartäre Ammoniumverbindungen, Antistatika, Bügelhilfsmittel, UV-Absorber, Antiredepositionsmittel, Germizide, antimikrobielle Wirkstoffe, Fungizide, Viskositätsregulatoren, Perlglanzgeber, Farbübertragungsinhibitoren, Einlaufverhinderer, Korrosionsinhibitoren, Konservierungsmittel, Weichmacher, Weichspüler, Proteinhydrolysate, Phobier- und Imprägniermittel, Hydrotrope, Silikonöle sowie Quell- und Schiebefestmittel enthält. Auch dies entspricht einer bevorzugten Ausführungsform der Erfindung.

Die erfindungsgemäßen partikelförmigen Träger sind dazu prädestiniert, in alle möglichen Wasch - oder Reinigungsmittel eingearbeitet zu werden.

Ein weiterer Gegenstand dieser Erfindung ist daher eine ein Detergenszusammensetzung, insbesondere ein Wasch- oder Reinigungsmittel, enthaltend:
o (A) erfindungsgemäßen partikelförmigen Träger, vorzugsweise in Mengen von 0,1-90 Gew.-%, vorteilhafterweise in Mengen von 1-70 Gew.-%, in vorteilhafterer Weise in Mengen von 2-60 %, in weiter vorteilhafter Weise in Mengen von 3-50 Gew.-%, in noch vorteilhafterer Weise 4-40 Gew.-%, in noch weiter vorteilhafterer Weise 5-30 Gew.-%, insbesondere 10-20 Gew.-% sowie
o (B) 0,01 Gew.-% bis 95 Gew.-%, vorzugsweise 5 Gew.-% bis 85 Gew.-%, vorteilhafterweise 3 Gew.-% bis 30 Gew.-%, insbesondere 5 Gew.-% bis 22 Gew.-% zusätzlicher Tensid(e).

Eine erfindungsgemäß bevorzugte Detergenszusammensetzung enthält neben den obligatorischen erfindungsgemäßen partikelförmigen Trägern z.B. die folgenden Bestandteile:
(a) Aniontenside, wie vorzugsweise Alkylbenzolsulfonat, Alkylsulfat, vorteilhafterweise in Mengen von 0-35 Gew.-%, beispielsweise > 0,1 Gew.-%, vorzugsweise 5 -10 Gew.-%, insbesondere 15 - 25 Gew.-%,
(b) Nichtionische Tenside, wie vorzugsweise Fettalkoholpolyglycolether, Alkylpolyglucosid, Fettsäureglucamid, vorteilhafterweise in Mengen von 0-30 Gew.-%, beispielsweise > 0,1 Gew.-%, vorzugsweise 2 -15 Gew.-%, insbesondere 4 -9 Gew.-%,
(c)Kationtenside, wie vorzugsweise quartäre Ammonium-Verbindungen, vorteilhafterweise in Mengen von 0-10 Gew.-%, vorzugsweise 0,1 -5 Gew.-%, insbesondere 0,2-4 Gew.-%,
(d) Gerüststoffe, wie vorzugsweise Zeolith, Polycarboxylat, Natriumcitrat, vorteilhafterweise in Mengen von 0-60 Gew.-%, beispielsweise > 0,1 Gew.-%, vorzugsweise 15 -55 Gew.-%, insbesondere 20 -40 Gew.-%,
(e) Alkalien, wie vorzugsweise Natriumcarbonat, vorteilhafterweise in Mengen von 0-30 Gew.-%, beispielsweise > 0,1 Gew.-%, vorzugsweise 2 -20 Gew.-%, insbesondere 5 -15 Gew.-%,
(f) Bleichmittel, wie vorzugsweise Natriumperborat, Natriumpercarbonat, vorteilhafterweise in Mengen von 0-30 Gew.-%, beispielsweise > 0,1 Gew.-%, vorzugsweise 10 -25Gew.-%, insbesondere 10 -20 Gew.-%,
(g) Korrosionsinhibitoren, wie vorzugsweise Natriumsilicat, vorteilhafterweise in Mengen von 0-20 Gew.-%, beispielsweise > 0,1 Gew.-%, vorzugsweise 1 -10 Gew.-%, insbesondere 2 -6 Gew.-%,
h) Stabilisatoren, wie vorzugsweise Phosphonate, vorteilhafterweise in Mengen von 0-10 Gew.-%, vorzugsweise 0,1 -5 Gew.-%, insbesondere 0,2 -1 Gew.-%,
(i) Schauminhibitor, wie vorzugsweise Seife, Siliconöle, Paraffine, vorteilhafterweise in Mengen von 0-10 Gew.-%, vorzugsweise 0,01-4 Gew.-%, insbesondere 0,1-2 Gew.-%,
(j) Enzyme, wie vorzugsweise Proteasen, Amylasen, Cellulasen, Lipasen, vorteilhafterweise in Mengen von 0-5 Gew.-%, vorzugsweise 0,1 -3 Gew.-%, insbesondere 0,2 -1 Gew.-%,
(k) Vergrauungsinhibitor, wie vorzugsweise Carboxymethyl-cellulose, vorteilhafterweise in Mengen von 0-5 Gew.-%, vorzugsweise 0,1-3 Gew.-%, insbesondere 0,2-1Gew.-%,
(l) Verfärbungsinhibitor, wie vorzugsweise Polyvinylpyrrolidon-(Derivate), vorteilhafterweise in Mengen von 0-3 Gew.-%, vorzugsweise 0,1-2 Gew.-%, insbesondere 0,2-1Gew.-%,
(m) Stellmittel, wie vorzugsweise Natriumsulfat, vorteilhafterweise in Mengen von 0-50 Gew.-%, beispielsweise > 0,1 Gew.-%, vorzugsweise 1-30 Gew.-%, insbesondere 5-20 Gew.-%,
(n) Riechstoffe, vorzugsweise in Mengen von 0-10 Gew.-%, beispielsweise > 0,1 Gew.-%, z.B. 1-5 Gew.-%,
(o) Optische Aufheller, wie z.B. Stilben-Derivate, Biphenyl-Derivate, vorzugsweise in Mengen von 0-3 Gew.-%, beispielsweise 0,1 -1Gew.-%, insbesondere 0,2 -0,5 Gew.-%,
(p) Wasser, z.B. in Mengen von 0-15 Gew.%, beispielsweise 1-10 Gew.-%.

Ein weiterer Gegenstand der vorliegenden Erfindung ist außerdem ein Verfahren zur Immobilisierung von Mikrokapseln, auf anorganischem Trägermaterial, wobei eine wässrige Suspension der Mikrokapseln, in einem Mischer mit einem übertrockneten anorganischen Träger gemischt wird, vorteilhafterweise bei einem niedrigen Energieeintrag.

Wie bereits zuvor dargelegt, läuft das Mischen dabei vorzugsweise bei Froude-Zahlen im Bereich von 0,1 bis 25, vorteilhafterweise von 0,5 bis 15, in weiter vorteilhafter Weise von 1 bis 10, in vorteilhafterer Weise von 1,5 bis 8, insbesondere von 2 bis 4 in gewünschter Weise ab. Die Froude-Zahl ist durch die Beziehung (w²· r)/g gegeben (w = Winkelgeschwindigkeit, r = Länge der Werkzeuge ab Mittelachse, g = Erdbeschleunigung). Das Arbeiten bei diesen Froude-Zahlen entspricht einer bevorzugten Ausführungsform der Erfindung.

Der bei dem Verfahren vorteilhafterweise eingesetzte übertrocknete anorganische Träger zeichnet sich gemäß einer bevorzugten Ausführungsform der Erfindung dadurch aus, dass das maximale theoretische Wasserbindevermögen des Trägers bzw. der Gesamtheit seiner Inhaltsstoffe um zumindest 10%, vorzugsweise zumindest 20 %, vorteilhafterweise um zumindest 30 %, insbesondere zumindest 40% oder gar 50% unterschritten ist.

Im übrigen gelten hier dieselben Ausführungen wie zuvor bei der Erläuterung der Herstellung des erfindungsgemäßen partikelförmigen Trägers kundgetan.

Ein weiterer Gegenstand der Erfindung ist ein Verfahren zum Aufbringen von Mikrokapseln, welche insbesondere bei thermischer, mechanischer, chemischer oder enzymatischer Einwirkung ihren Inhalt freisetzen, auf Textilien durch Behandlung dieser Textilien in einem Textilbehandlungsbad, zu welchem man partikelförmige Träger, wie sie zuvor beschrieben wurden, gibt. Wenn das Verfahren unter Verwendung einer automatischen Waschmaschine durchgeführt wird, so liegt eine bevorzugte Ausführungsform der Erfindung vor.

Ein weiterer Gegenstand der Erfindung ist ein Verfahren zum Beduften von Textilien, bei dem man in einem ersten Schritt von einem Verfahren zum Aufbringen von Mikrokapseln, welche insbesondere bei thermischer, mechanischer, chemischer oder enzymatischer Einwirkung ihren Inhalt freisetzen, auf Textilien durch Behandlung dieser Textilien in einem Textilbehandlungsbad, zu welchem man partikelförmige Träger, wie sie zuvor beschrieben wurden, gibt, Gebrauch macht, und in einem zweiten Schritt, den Inhalt der aufgebrachten Mikrokapseln, die Riechstoffe enthalten, unmittelbar auf dem Textil freisetzt und zwar durch eine thermische, mechanische, chemische oder enzymatische Einwirkung, insbesondere durch Bügeln, Trocknen in einem Wäschetrockner oder manuelles Reiben oder Rubbeln des Textils.

Im folgenden werden in nicht beschränkender Weise einige bevorzugt einsetzbare, jedoch rein optionale Inhaltsstoffe von Wasch- oder Reinigungsmitteln näher beschrieben, welche vorteilhafterweise in den partikelförmigen Trägern als solchen und/oder in den feinpartikulären Wirkstoffträgermatrices und/oder in den Wasch- oder Reinigungsmitteln, in welchen die erfindungsemäßen partikelförmige Träger enthalten sind, optional enthalten sein können.

Zu diesen optionalen Inhaltsstoffen gehören die Gerüststoffe. Zu den Gerüststoffe zählen insbesondere die Zeolithe, Silikate, Carbonate, organische Cobuilder und -wo keine ökologischen Vorurteile gegen ihren Einsatz bestehen- auch die Phosphate.

Der einsetzbare feinkristalline, synthetische Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

n Na₂O · (1-n) K₂O · Al₂O₃ · (2 - 2,5) SiO₂ · (3,5 - 5,5) H₂O

beschrieben werden kann. Geeignete Zeolithe weisen eine mittlere Teilchengröße von vorzugsweise weniger als 10 µm (Volumenverteilung; Meßmethode: Coulter Counter) auf.

Geeignete kristalline, schichtförmige Natriumsilikate besitzen die allgemeine Formel NaMSiₓO₂ₓ₊₁ H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl ß- als auch δ-Natriumdisilikate Na₂Si₂O₅ · y H₂O bevorzugt.

Im Rahmen der vorliegenden Erfindung kann es bevorzugt sein, dass der partikelförmige Träger Silikate enthält, vorzugsweise Alkalisilikate, besonders bevorzugt kristalline oder amorphe Alkalidisilikate enthalten, z.B. in Mengen von 10 bis 60 Gew.-%, vorzugsweise von 15 bis 50 Gew.-% und insbesondere von 20 bis 40 Gew.-%, jeweils bezogen auf das Gewicht des gesamten partikelförmigen Trägers. Wenn vom Gewicht des gesamten partikelförmigen Trägers oder vom Gesamtgewicht des partikelförmigen Trägers die Rede ist, so ist damit jeweils das Gewicht des partikelförmigen Trägers inklusive der feinpartikulären Wirkstoffträgermatrices gemeint, es sei denn, es wird etwas anderes angegeben.

Weitere optionale Gerüststoffe sind die Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden. Besonders bevorzugt ist eine Mischung aus Tripolyphosphat und Natriumcarbonat. Ebenfalls besonders bevorzugt ist eine Mischung aus Tripolyphosphat und Natriumcarbonat und Natriumdisilikat.

Aufgrund ihrer im Vergleich mit anderen Buildersubstanzen geringen chemischen Kompatibilität mit anderen Inhaltsstoffen werden die Alkalimetallhydroxide, wenn überhaupt, bevorzugt nur in geringen Mengen, vorzugsweise in Mengen unterhalb 10 Gew.-%, bevorzugt unterhalb 6 Gew.-%, besonders bevorzugt unterhalb 4 Gew.-% und insbesondere unterhalb 2 Gew.-%, jeweils bezogen auf das Gesamtgewicht des partikelförmigen Trägers, eingesetzt. Besonders bevorzugt werden partikelförmige Träger, welche bezogen auf ihr Gesamtgewicht weniger als 0,5 Gew.-% und insbesondere keine Alkalimetallhydroxide enthalten.

Besonders bevorzugt kann der Einsatz von Carbonat(en) und/oder Hydrogencarbonat(en), vorzugsweise Alkalicarbonat(en), besonders bevorzugt Natriumcarbonat, z.B. in Mengen von 2 bis 50 Gew.-%, vorzugsweise von 5 bis 40 Gew.-% und insbesondere von 7,5 bis 30 Gew.-%, jeweils bezogen auf das Gesamtgewicht der partikelförmigen Träger sein. Besonders bevorzugt können partikelförmige Träger sein, welche bezogen auf ihr Gesamtgewicht weniger als 20 Gew.-%, vorzugsweise weniger als 17 Gew.-%, bevorzugt weniger als 13 Gew.-% und insbesondere weniger als 9 Gew.-% Carbonat(e) und/oder Hydrogencarbonat(e), vorzugsweise Alkalicarbonat(e), besonders bevorzugt Natriumcarbonat enthalten.

Als optionale organische Cobuilder sind insbesondere Polycarboxylate/Polycarbonsäuren, polymere Polycarboxylate, Asparaginsäure, Polyacetale, Dextrine, weitere organische Cobuilder (siehe unten) sowie Phosphonate zu nennen.

Der Gehalt an optionalen (co-)polymeren Polycarboxylaten kann in den partikelförmigen Trägern vorzugsweise 0,5 bis 20 Gew.-%, insbesondere 3 bis 10 Gew.-% betragen, bezogen auf ihr Gesamtgewicht.

Alle vorgenannten Gerüststoffe bzw. Buildersubstanzen können optional in den partikelförmigen Trägern enthalten sein. Die partikelförmigen Träger können optional auch frei davon sein.

Zur Gruppe der Tenside, welche optional in den partikelförmigen Trägern enthalten sein können, werden insbesondere die nichtionischen, die anionischen, die kationischen und die amphoteren Tenside gezählt.

Als optionale anionische Tenside können beispielsweise solche vom Typ der Sulfonate und Sulfate eingesetzt werden. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos- , Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Alle vorgenannten anionischen Tenside sind nur optional. Insbesondere alle vorgenannten anionischen Tenside können optional in den partikelförmigen Trägern enthalten sein. Die partikelförmigen Träger können optional auch frei davon sein.

Der Aniontensidgehalt erfindungsgemäßer partikelförmiger Träger kann z.B. im Bereich von vorzugsweise 1-60 Gw.-% vorteilhafterweise 5-40 Gew.-%, insbesondere 10-30 Gew.-% liegen, bezogen auf ihr Gesamtgewicht.

An Stelle der genannten Tenside oder auch in Verbindung mit ihnen können optional auch kationische und/oder amphotere Tenside eingesetzt werden. Die partikelförmigen Träger können optional auch frei davon sein.

Der Gehalt an kationischen und/oder amphoteren Tensiden in erfindungsgemäßen partikelförmigen Trägern kann vorzugsweise weniger als 10 Gew.-%, bevorzugt weniger als 5 Gew.-%, ganz besonders bevorzugt weniger als 2 Gew.-% und insbesondere weniger als 1 Gew.-% betragen, bezogen auf ihr Gesamtgewicht. Es kann auch bevorzugt sein, dass keine kationischen oder amphoteren Tenside enthalten sind.

Zur Gruppe solcher Polymere, die in den partikelförmigen Trägern optional enthalten sein können, zählen insbesondere die wasch- oder reinigungsaktiven Poylmere, beispielsweise die als Enthärter wirksamen Polymere.

Bleichmittel sind eine mit besonderem Vorzug optional in den partikelförmigen Trägern enthaltene wasch- oder reinigungsaktive Substanz. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxypyrophosphate, Citratperhy-drate sowie H₂O₂ liefernde persaure Salze oder Persäuren, wie Perbenzoate, Peroxophthalate, Diperazelainsäure, Phthaloiminopersäure oder Diperdodecandisäure.

Weiterhin können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesiummonoperphthalat, (b) die aliphatischen oder substituiert aliphatischen Peroxysäuren, wie Peroxylaurinsäure, Peroxystearinsäure, ε-Phthalimidoperoxycapronsäure [Phthaliminoperoxyhexansäure (PAP)], o-Carboxybenzamidoperoxycapronsäure, N-Nonenylamidoperadipinsäure und N-Nonenylamidopersuccinate, und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthatoyl-di(6-aminopercapronsäue) können eingesetzt werden.

Als Bleichmittel können auch Chlor oder Brom freisetzende Substanzen eingesetzt werden. Unter den geeigneten Chlor oder Brom freisetzenden Materialien kommen beispielsweise heterozyklische N-Brom- und N-Chloramide, beispielsweise Trichlorisocyanursäure, Tribromisocyanursäure, Dibromisocyanur-säure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Insbesondere die vorgenannten Bleichmittel können optional in den partikelförmigen Trägern enthalten sein. Die partikelförmigen Träger können optional auch frei davon sein.

Erfindungsgemäß werden solche Wasch- oder Reinigungsmittel bevorzugt, die 1 bis 35 Gew.-%, vorzugsweise 2,5 bis 30 Gew.-%, besonders bevorzugt 3,5 bis 20 Gew.-% und insbesondere 5 bis 15 Gew.-% Bleichmittel, vorzugsweise Natriumpercarbonat, enthalten, bezogen auf das gesamte Wasch- oder Reinigungsmittel.

Bleichaktivatoren können in den erfindungsgemäßen partikelförmigen Trägern beispielsweise eingesetzt werden, um beim Reinigen bei Temperaturen von 60 °C und darunter eine verbesserte Bleichwirkung zu erreichen. Als optionale Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-tri-azin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur Steigerung der Wasch-, beziehungsweise Reinigungsleistung sind Enzyme optional einsetzbar. Hierzu gehören insbesondere Proteasen, Amylasen, Lipasen, Hemicellulasen, Cellulasen oder Oxidoreduktasen, sowie vorzugsweise deren Gemische. Diese Enzyme sind im Prinzip natürlichen Ursprungs; ausgehend von den natürlichen Molekülen stehen insbesondere für den Einsatz in Zusammenhang mit Wasch- und Reinigungsvorgängen verbesserte Varianten zur Verfügung, die entsprechend bevorzugt eingesetzt werden.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsvaerd, Dänemark, erhältlich. Die Subtilisine 147 und 309 werden unter den Handelsnamen Esperase^{®}, beziehungsweise Savinase^{®} von der Firma Novozymes vertrieben. Von der Protease aus *Bacillus lentus* DSM 5483 leiten sich die unter der Bezeichnung BLAP^{®} geführten Varianten ab.

Weitere brauchbare Proteasen sind beispielsweise die unter den Handelsnamen Durazym^{®}, Relase^{®}, Everlase^{®}, Nafizym, Natalase^{®}, Kannase^{®} und Ovozymes^{®} von der Firma Novozymes, die unter den Handelsnamen, Purafect^{®}, Purafecf^{®} OxP und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme.

Beispiele für erfindungsgemäß einsetzbare Amylasen sind die α-Amylasen aus *Bacillus licheniformis,* aus *B. amyloliquefaciens* oder aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl^{®} und von der Firma Genencor unter dem Namen Purastar^{®}ST erhältlich. Weiterentwicklungsprodukte dieser α-Amylase sind von der Firma Novozymes unter den Handelsnamen Duramyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von *B. amyloliquefaciens* wird von der Firma Novozymes unter dem Namen BAN^{®} vertrieben, und abgeleitete Varianten von der α-Amylase aus *B. stearothermophilus* unter den Namen BSG^{®} und Novamyl^{®}, ebenfalls von der Firma Novozymes.

Desweiteren sind für diesen Zweck die α-Amylase aus *Bacillus sp.* A 7-7 (DSM 12368) und die Cyclodextrin-Glucanotransferase (CGTase) aus *B. agaradherens* (DSM 9948) hervorzuheben.

Darüber hinaus sind die unter den Handelsnamen Fungamyl^{®} von der Firma Novozymes erhältlichen Weiterentwicklungen der α-Amylase aus *Aspergillus niger* und *A*. *oryzae* geeignet. Ein weiteres Handelsprodukt ist beispielsweise die Amylase-LT^{®}.

Erfindungsgemäß einsetzbar sind weiterhin Lipasen oder Cutinasen, insbesondere wegen ihrer Triglycerid-spaltenden Aktivitäten, aber auch, um aus geeigneten Vorstufen *in situ* Persäuren zu erzeugen. Hierzu gehören beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Desweiteren sind beispielsweise die Cutinasen einsetzbar, die ursprünglich aus *Fusarium solani pisi* und *Humicola insolens* isoliert worden sind. Ebenso brauchbare Lipasen sind von der Firma Amano unter den Bezeichnungen Lipase CE^{®}, Lipase P^{®}, Lipase B^{®}, beziehungsweise Lipase CES^{®}, Lipase AKG^{®}, Bacillis sp. Lipase^{®}, Lipase AP^{®}, Lipase M-AP^{®} und Lipase AML^{®} erhältlich. Von der Firma Genencor sind beispielsweise die Lipasen, beziehungsweise Cutinasen einsetzbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Genencor.

Weiterhin können Enzyme eingesetzt werden, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen. Geeignete Mannanasen sind beispielsweise unter den Namen Gamanase^{®} und Pektinex AR^{®} von der Firma Novozymes, unter dem Namen Rohapec^{®} B1L von der Firma AB Enzymes und unter dem Namen Pyrolase^{®} von der Firma Diversa Corp., San Diego, CA, USA erhältlich. Die aus *B. subtilis* gewonnene β-Glucanase ist unter dem Namen Cereflo^{®} von der Firma Novozymes erhältlich.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose - oder Mangan-peroxidasen, Dioxygenasen oder Laccasen (Phenoloxidasen, Polyphenoloxidasen) eingesetzt werden. Als geeignete Handelsprodukte sind Denilite^{®} 1 und 2 der Firma Novozymes zu nennen. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Die Enzyme stammen beispielsweise entweder ursprünglich aus Mikroorganismen, etwa der Gattungen *Bacillus, Streptomyces, Humicola,* oder *Pseudomonas,* und/oder werden nach an sich bekannten biotechnologischen Verfahren durch geeignete Mikroorganismen produziert, etwa durch transgene Expressionswirte der Gattungen *Bacillus* oder filamentöse Fungi.

Die Aufreinigung der betreffenden Enzyme erfolgt vorzugsweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte.

Die Enzyme können in jeder nach dem Stand der Technik etablierten Form eingesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt.

Ein Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Zu diesem Zweck können Stabilisatoren enthalten sein; die Bereitstellung derartiger Mittel stellt eine bevorzugte Ausführungsform der vorliegenden Erfindung dar.

Eine Gruppe von Stabilisatoren sind reversible Proteaseinhibitoren. Häufig werden Benzamidin-Hydro-chlorid, Borax, Borsäuren, Boronsäuren oder deren Salze oder Ester verwendet, darunter vor allem Derivate mit aromatischen Gruppen, etwa ortho-substituierte, meta-substituierte und para-substituierte Phenylboronsäuren, beziehungsweise deren Salze oder Ester. Als peptidische Proteaseinhibitoren sind unter anderem Ovomucoid und Leupeptin zu erwähnen; eine zusätzliche Option ist die Bildung von Fusionsproteinen aus Proteasen und Peptid-Inhibitoren.

Weitere Enzymstabilisatoren sind Aminoalkohole wie Mono-, Di-, Triethanol- und -Propanolamin und deren Mischungen, aliphatische Carbonsäuren bis zu C₁₂, wie Bernsteinsäure, andere Dicarbonsäuren oder Salze der genannten Säuren. Auch endgruppenverschlossene Fettsäureamidalkoxylate sind geeignet. Bestimmte als Builder eingesetzte organische Säuren vermögen zusätzlich ein enthaltenes Enzym zu stabilisieren.

Niedere aliphatische Alkohole, vor allem aber Polyole, wie beispielsweise Glycerin, Ethylenglykol, Pro-pylenglykol oder Sorbit sind weitere häufig eingesetzte Enzymstabilisatoren. Ebenso werden Calciumsalze verwendet, wie beispielsweise Calcium-Acetat oder Calcium-Formiat, und Magnesiumsalze.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthalten-de Polymere wirken als Enzymstabilisatoren. Andere polymere Stabilisatoren sind die linearen C₈-C₁₈ Polyoxyalkylene. Alkylpolyglycoside können die enzymatischen Komponenten stabilisieren und sogar in ihrer Leistung steigern. Vernetzte N-haltige Verbindungen wirken ebenfalls als Enzym-Stabilisatoren.

Reduktionsmittel und Antioxidantien erhöhen die Stabilität der Enzyme gegenüber oxidativem Zerfall. Ein schwefelhaltiges Reduktionsmittel ist beispielsweise Natrium-Sulfit.

Bevorzugt werden Kombinationen von Stabilisatoren verwendet, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird durch die Kombination mit Borsäure und/oder Borsäurederivaten und Polyolen gesteigert und durch die zusätzliche Verwendung von zweiwertigen Kationen, wie zum Beispiel Calcium-Ionen weiter verstärkt.

Die partikelförmigen Träger können angefärbt sein. Bevorzugte optionale Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber den mit den farbstoffhaltigen Mitteln zu behandelnden Substraten wie beispielsweise Textilien, Glas, Keramik oder Kunststoffgeschirr, um diese nicht anzufärben.

Die zuvor beschriebenen Inhaltsstoffe von Wasch- oder Reinigungsmitteln sind alle rein optional. Sie können vorteilhafterweise in den partikelförmigen Trägern als solchen optional enthalten sein und/oder in den feinpartikulären Wirkstoffträgermatrices und/oder in den Wasch- oder Reinigungsmitteln, in welchen die erfindungsemäßen partikelförmige Träger enthalten sind.

Es ist auch möglich, dass die partikelförmigen Trägern als solche und/die feinpartikulären Wirkstoffträgermatrices und/oder die Wasch- oder Reinigungsmitteln, in welchen die erfindungsemäßen partikelförmige Träger enthalten sind, frei sind von den zuvor beschriebenen optionalen Inhaltsstoffen, z.B. frei sind von einzelnen oder jedem einzelnen der zuvor beschriebenen optionalen Inhaltsstoffe.

Zusätzlich zu den bisher ausführlich beschriebenen Komponenten, aber auch unabhängig von diesen, können die partikelförmigen Träger und/oder die feinpartikulären Wirkstoffträgermatrices und/oder die Wasch- oder Reinigungsmitteln, welche die erfindungsemäßen partikelförmige Träger enthalten, optional weitere Inhaltsstoffe enthalten, welche insbesondere die anwendungstechnischen und/oder ästhetischen Eigenschaften dieser Mittel weiter verbessern.

Es können demnach optional ein oder mehrere Stoffe aus der Gruppe der Elektrolyte, pH-Stellmittel, Fluoreszenzmittel, Hydrotope, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffen, Germizide, Fungizide, Antioxidantien, Antistatika, Bügelhilfsmittel, Phobier- und Imprägniermittel, Quell- und Schiebefestmittel sowie UV-Absorber enthalten sein.

Als Elektrolyte aus der Gruppe der anorganischen Salze kann optional eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ bevorzugt.

Um den pH-Wert in den gewünschten Bereich zu bringen, kann der Einsatz von pH-Stellmitteln optional angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet.

Zur Pflege der Textilien und zur Verbesserung der Textileigenschaften wie einem weicheren "Griff" (Avivage) und verringerter elektrostatischer Aufladung (erhöhter Tragekomfort) können optional Weichspüler eingesetzt werden. Die Wirkstoffe in Weichspülformulierungen sind in der Regel "Esterquats", also quartäre Ammoniumverbindungen mit zwei hydrophoben Resten, wie beispielsweise das Disteraryldimethylammoniumchlorid, welches jedoch wegen seiner ungenügenden biologischen Abbaubarkeit vorzugsweise durch quartäre Ammoniumverbindungen ersetzt wird, die in ihren hydrophoben Resten Estergruppen als Sollbruchstellen für den biologischen Abbau enthalten.

Derartige "Esterquats" mit verbesserter biologischer Abbaubarkeit sind beispielsweise dadurch erhältlich, dass man Mischungen von Methyldiethanolamin und/oder Triethanolamin mit Fettsäuren verestert und die Reaktionsprodukte anschließend in an sich bekannter Weise mit Alkylierungsmitteln quatemiert. Als Appretur weiterhin geeignet ist Dimethylolethylenharnstoff.

### Beispiel

Folgender Träger wurde unter übertrocknenden Bedingungen durch Sprühtrocknung hergestellt:

| | |
|---|---|
| Polycarboxylat | 5 Gew.-% |
| Ethoxylierter Fettalkohol | 0,5 Gew.% |
| Zeolith A (wasserfreie Aktivsubstanz) | 15 Gew.-% |
| Carboxymethylcellulose-Na.-Salz | 2 Gew.-% |
| Soda und Na₂SO₄ | 73 Gew.-% |
| Wasser | 4 Gew.-% |
| Rest (Salze, Verunreinigungen) | 0,5 Gew.-% |
| Summe | 100 Gew-% |

Der Träger hatte also einen Gesamtwassergehalt von nur 4 Gew.-%, bezogen auf den gesamten Träger.

Dieser Träger wurde anschließend mit einer wässrigen Suspension aus Melamin-Formaldehyd-Mikrokapseln, die ein Parfüm enthielten, in einem Paddelmischer für einige Minuten bei niedrigem Energieeintrag so vermischt, dass das Wasser von dem übertrockneten Träger aufgenommen wurde.

Es resultierte ein erfindungsgemäßer partikelförmiger Träger, welcher teilweise mit den Mikrokapseln belegt war. Die Mikrokapseln waren auf dem Träger fest immobilisiert und außerdem immer noch unversehrt, d.h. enthielten das Parfüm.

Der erfindungsgemäße partikelförmige Träger wurde anschließend nochmal mit einem weiteren Parfümöl beduftet.

Der resultierende erfindungsgemäße partikelförmige Träger hatte danach folgende Gesamtzsammensetzung:

| | |
|---|---|
| Träger | 80 Gew.-% |
| Mikrokapseln | 9 Gew.-% |
| Weiteres Parfüm | 11 Gew.-% |

Mikroskopische Aufnahme zeigten die Fixierung der Mikrokapseln auf dem anorganischen Träger.

Der erfindungsgemäße partikelförmige Träger wies ein Schüttgewicht von 580 g/L auf.

Der erfindungsgemäße partikelförmige Träger wurde anschließend mit einer gewöhnlichen pulverförmigen, parfümfreien Waschmittelmatrix vermengt, so dass ein vollwertiges, parfümiertes pulverförmiges Waschmittel resultierte.

Dieses Waschmittel führte bei Einsatz in einer gewöhnlichen automatischen Waschmaschine zu einem Waschgut, auf dem sich Melamin-Formaldehyd-Mikrokapseln niedergeschlagen hatten. Diese Mikrokapseln konnten sich z.B. durch manuelles Rubbeln öffnen lassen, so dass der in den Mikrokapseln enthaltene Duft dann freigetzt wurde und ein Frischeeindruck erzeugt werden konnte.

## Patentansprüche

1. Partikelförmiger Träger, **dadurch gekennzeichnet, dass** er zumindest teilweise mit Mikrokapseln, enthaltend Riechstoffe , belegt ist

2. Träger nach Anspruch 1, **dadurch gekennzeichnet, dass** er anorganisches Tragermaterial umfasst, vorzugsweise ausgewählt aus der Gruppe Zeolithe, Sulfate, Carbonate, Silikate, Tone, Kieselsäure und/oder deren Gemische.

3. Träger gemäß einem der vorigen Ansprüche 1 oder 2, **dadurch gekennzeichnet, dass** er mit einer Flüssigkeit imprägniert ist, vorzugsweise umfassend
i. Riechstoffe (Parfümöle)
ii. flüssige Wasch- und Reinigungsmittelinhaltsstoffe, wie vorzugsweise Tenside, insbesondere Niotenside, Silikonöle, Paraffine
iii. flüssige Kosmetikinhaltstoffe, wie vorzugsweise Öle
iv. flüssige nicht-pharmazeutische Additive oder Wirkstoffe und/oder Mischungen vorgenannter,
wobei die Flüssigkeit insbesondere zwischen 0,1 und 30 Gew.-% des Gesamtgewichtes des Trägers ausmacht.

4. Träger gemäß einem der vorigen Ansprüche 1 bis 3, **dadurch gekennzeichnet, dass** das Kapselmaterial der Mikrokapseln ausgewählt ist aus
(a) Homopolymeren, vorzugsweise
- durch Polymerisation von Vinyl-Gruppen-haltigen Monomeren zugängliche Polymere, wie z.B. Polyvinylacetate, Polyvinylalkohol, Polyvinylpyrrolidon,
- Polycarboxylate, Polycarbonsäuren, z.B. Polyacrylsäure, Polymethacrylsäure,
- Polysulfonsäuren, z.B. Polystyrolsulfonsäure,
- Polyester, z.b. Polyacrylate, Glycolpolyacrylat.
- Polyamide, z.B. Polyacrylamide,
- Polyurethane,
- Polyethylenoxide, Polypropylenoxide oder ander Polyalkylenglycolderivative;
(b) Polykondensate, vorzugsweise
- ethoxylierte Phenol-Formaldehyd-Harze
- sulfonierte aromatische Formaldehyd-Harze
- Harnstoff oder Melamin- Formaldehyd-Harze, z.B. Melamin-Harnstoff-Formaldehyd-Harze, Melamin-Phenol-Formaldehyd-Harze
- Polyamid-Harze, Polyamin-Harze, und Epoxid-Harze
(c) AB-Copolymere vorzugsweise
- Styrolcopolymere, z.B. Styrol-Acrylsäure-Polymere oderr styrol-Ethylenoxid-Polymere
- Copolymer von Polyvinyl- und Maleinsäure-Compounds, z.B. Styrol-Maleinsäureanhydrid-Polymer
- Polyvinyl-Polyalkylen-Ccopolymere, z.B. Vinylacetat
- Ethylenpolymer, Ethylen-Acrylsäure-Acrylsäureester-Polymere oder Ethylen-Acrylsäure-Acrylonitril-Polymere
- andere Vinylcopolymere, z.B. Vinylacetatpolymere, Acrylsäure-Acrylonitril-Polymere, Acrylsäure-Acrylamid-Polymere;
(d) ABA-Blockcopolymer, wobei vorzugsweise
- "A" für wasserlösliche oder wasserquellbare Gruppen wie Polyethylenoxid, Polyvinylalkohol, Polyacrylamid, Polyacrylsäure, Poly vinylpyrrolidon oder Polyccaprolacton,
- "B" für weniger oder kaum wasserlösliche Gruppen wie Polypropylenoxid, Polyvinylacetat, Polyvinylbutyral, Polylaurylmethacrylat, Polystyrol, Polyhydroxystearinsäure, Polysiloxan steht
(e) B(A)ₙ Pfropf(co)polymere, wobei vorzugsweise
- "A" für wasserlösliche oder wasserquellbare Gruppen wie Vinylalkohol, Vinylacetate, Ethylenoxide, Propylenoxid, Vinylsulphonate, Acrylsäuren und Vinylamine, und
- "B" für Vinylpolymer-Ketten oder Siloxan-Ketten steht,
(f) natürliche oder abgewandelte natürliche Polymere z.B. Cellulosederivative, wie Carboxymethylcellulose, Hydroxypropylmethylcellulose,
Methylcellulose, Ethylcellulose, Celluloseacetat, Cellulosenitrat, Eiweißverbindungen (Gelatine, Albumin, Casein).

5. Träger gemäß einem der vorigen Ansprüche 1 bis 4, **dadurch gekennzeichnet, dass** der mit den Mikrokapseln belegte Träger außerdem beschichtet ist, vorzugsweise abgepudert und/oder mit einem Film gecoatet.

6. Träger nach einem der vorigen Ansprüche 1 bis 5, erhältlich durch Mischen einer wäßrigen Suspension von parfümhaltigen Mikrokapseln mit einem übertrockneten anorganischen Träger in einem Mischer mit niedrigem Energieeintrag, vorzugsweise Paddelmischer.

7. Träger nach dem vorigen Anspruch 6. **dadurch gekennzeichnet, dass** der übertrocknete anorganische Träger einen Wassergehalt < 10 Gew.-%, vorzugsweise < 8, vorteilhafterweise < 8, in noch vorteilhafterweise < 5 aufweist, bezogen auf den eingesetzten übertrockneten anorganischen Träger.

8. Träger nach einem der vorigen Ansprüche 6 oder 7, **dadurch gekennzeichnet, dass** die wässrige Suspension von parfümhaltigen Mikrokapseln zumindest 30 Gew.-%, vorzugsweise zumindest 40 Gew.-%, insbesondere zumindest 50 Gew.-% Mikrokapseln enthält.

9. Detergenszusammensetzung, enthaltend:
o (A) Träger nach einem der Ansprüche der Ansprüche 1-8,
o (B) 0, 01 Gew.-% bis 95 Gew.-% zusätzlicher Tensid(e).

10. Verfahren zur Immobilisierung von parfümhaltigen Mikrokapseln auf anorganischem Trägermaterial, **dadurch gekennzeichnet, dass** eine wässrige Suspension von parfümhaltigen Mikrokapseln in einem Mischer mit einem übertrockneten anorganischen Träger gemischt wird.

11. Verfahren nach Anspruch 10, wobei in dem übertrockneten anorganischen Träger das maximale theoretische Wasserbindevermögen des Trägers bzw. der Gesamtheit seiner Inhaltsstoffe um zumindest 10%, vorzugsweise zumindest 20 %, insbesondere zumindest 30% unterschritten ist.

12. Verfahren zum Aufbringen von parfümhaltigen Mikrokapseln, welche bei thermischer, mechanischer, chemischer oder enzymatischer Einwirkung ihren Inhalt freisetzen, auf Textilien durch Behandlung dieser Textilien in einem Textilbehandlungsbad, zu welchem man partikelförmige Träger nach einem der Ansprüche 1-8 oder eine Detergenszusammensetzung nach Anspruch 9 gibt.

## Claims

1. A particulate carrier, **characterised in that** it is at least in part covered with microcapsules containing fragrances.

2. A carrier according to claim 1, **characterised in that** it comprises inorganic carrier material, preferably selected from the group of zeolites, sulfates, carbonates, silicates, clays, silica and/or mixtures thereof.

3. A carrier according to either one of claims 1 or 2 above, **characterised in that** it is impregnated with a liquid preferably comprising
i. fragrances (perfume oils)
ii. liquid washing and cleaning agent ingredients, such as preferably surfactants, in particular nonionic surfactants, silicone oils, paraffins,
iii. liquid cosmetic ingredients, such as preferably oils
iv. liquid non-pharmaceutical additives or active ingredients and/or mixtures thereof,
wherein the liquid in particular constitutes between 0.1 and 30 wt.% of the total weight of the carrier.

4. A carrier according to any one of claims 1 to 3 above, **characterised in that** the capsule material of the microcapsules is selected from
(a) homopolymers, preferably
- polymers obtainable by polymerising monomers containing vinyl groups, such as for example polyvinyl acetates, polyvinyl alcohol, polyvinylpyrrolidone,
- polycarboxylates, polycarboxylic acids, for example polyacrylic acid, polymethacrylic acid,
- polysulfonic acids, for example polystyrenesulfonic acid,
- polyesters, for example polyacrylates, glycol polyacrylate,
- polyamides, for example polyacrylamides,
- polyurethanes,
- polyethylene oxides, polypropylene oxides or other polyalkylene glycol derivatives;
(b) polycondensation products, preferably
- ethoxylated phenol-formaldehyde resins
- sulfonated aromatic formaldehyde resins
- urea or melamine-formaldehyde resins, for example melamine-urea-formaldehyde resins, melamine-phenol-formaldehyde resins
- polyamide resins, polyamine resins and epoxy resins
(c) AB copolymers, preferably
- styrene copolymers, for example styrene-acrylic acid polymers or styrene-ethylene oxide polymers
- copolymers of polyvinyl and maleic acid compounds, for example styrene-maleic anhydride polymer
- polyvinyl-polyalkylene copolymers, for example vinyl acetate
- ethylene polymers, ethylene-acrylic acid-acrylic acid ester polymers or ethylene-acrylic acid-acrylonitrile polymers
- other vinyl copolymers, for example vinyl acetate polymers, acrylic acid-acrylonitrile polymers, acrylic acid-acrylamide polymers;
(d) ABA block copolymers, wherein preferably
- "A" denotes water-soluble or water-swellable groups such as polyethylene oxide, polyvinyl alcohol, polyacrylamide, polyacrylic acid, polyvinylpyrrolidone or polycaprolactone,
- "B" denotes less or sparingly water-soluble groups such as polypropylene oxide, polyvinyl acetate, polyvinyl butyral, polylauryl methacrylate, polystyrene, polyhydroxystearic acid, polysiloxane
(e) B(A)ₙ graft (co)polymers, wherein preferably
- "A" denotes water-soluble or water-swellable groups such as vinyl alcohol, vinyl acetates, ethylene oxides, propylene oxide, vinylsulfonates, acrylic acids and vinylamines, and
- "B" denotes vinyl polymer chains or siloxane chains,
(f) natural or modified natural polymers, for example cellulose derivatives, such as carboxymethylcellulose, hydroxypropylmethylcellulose, methylcellulose, ethylcellulose, cellulose acetate, cellulose nitrate, protein compounds (gelatin, albumin, casein).

5. A carrier according to any of claims 1 to 4 above, **characterised in that** the carrier covered with surface-modified with microcapsules is moreover coated, preferably powder-finished and/or film-coated.

6. A carrier according to any one of claims 1 to 5 above, obtainable by mixing an aqueous suspension of perfume-containing microcapsules with an overdried inorganic carrier in a mixer with a low energy input, preferably a paddle mixer.

7. A carrier according to claim 6 above, **characterised in that** the overdried inorganic carrier has a water content of < 10 wt.%, preferably of < 8, advantageously of < 6, still more advantageously of < 5, relative to the introduced overdried inorganic carrier.

8. A carrier according to either one of claims 6 or 7 above, **characterised in that** the aqueous suspension of perfume-containing microcapsules contains at least 30 wt.%, preferably at least 40 wt.%, in particular at least 50 wt.% of microcapsules.

9. A detergent composition containing:
○ (A) a carrier according to any one of claims 1-8,
○ (B) 0.01 wt.% to 95 wt.% of additional surfactant(s).

10. A method for immobilising perfume-containing microcapsules on an inorganic carrier material, **characterised in that** an aqueous suspension of perfume-containing microcapsules is mixed in a mixer with an overdried inorganic carrier.

11. A method according to claim 10, wherein the water-binding capacity of the overdried inorganic carrier is at least 10%, preferably at least 20%, in particular at least 30% below the maximum theoretical water-binding capacity of the carrier or of the entirety of the ingredients thereof.

12. A method for applying perfume-containing microcapsules, which release their contents on exposure to thermal, mechanical, chemical or enzymatic action, onto textiles by treating said textiles in a textile treatment bath to which are added particulate carriers according to any one of claims 1-8 or a detergent composition according to claim 9.

## Revendications

1. Support particulaire, **caractérisé en ce qu'**il est recouvert au moins en partie avec des microcapsules contenant des fragrances.

2. Support selon la revendication 1, **caractérisé en ce qu'**il comprend une matière de support inorganique, de préférence choisie parmi le groupe des zéolithes, des sulfates, des carbonates, des silicates, des argiles, de l'acide silicique et/ou de leurs mélanges.

3. Support selon l'une quelconque des revendications précédentes 1 ou 2, **caractérisé en ce qu'**il est imprégné avec un liquide comprenant de préférence :
i. des fragrances (huiles de parfums) ;
ii. des constituants d'agents de lavage et de nettoyage liquides, tels que, à titre préférentiel, des agents tensioactifs, en particulier des agents tensioactifs non ioniques, des huiles de silicone, des paraffines ;
iii. des constituants de cosmétiques liquides, tels que, à titre préférentiel, des huiles ;
iv. des additifs ou des substances actives non pharmaceutiques liquides ; et/ou
des mélanges des éléments susmentionnés,
le liquide représentant en particulier entre 0,1 et 30 % en poids du poids total du support.

4. Support selon l'une quelconque des revendications précédentes 1 à 3, **caractérisé en ce que** la matière d'encapsulage des microcapsules est choisie parmi :
(a) des homopolymères, à titre préférentiel :
- des polymères que l'on obtient par polymérisation de monomères contenant des groupes vinyle, comme par exemple des acétates de polyvinyle, l'alcool polyvinylique, la polyvinylpyrolidone ;
- des polycarboxylates, des acides polycarboxyliques, par exemple l'acide polyacrylique, l'acide polyméthacrylique ;
- des acides polysulfoniques, par exemple l'acide polystyrène-sulfonique ;
- des polyesters, par exemple des polyacrylates, le polyacrylate de glycol ;
- des polyamides, par exemple des polyacrylamides ;
- des polyuréthanes ;
- des oxydes de polyéthylène, des oxydes de polypropylène ou d'autres dérivés de polyalkylèneglycols ;
(b) des polycondensats, à titre préférentiel :
- des résines de phénol-formaldéhyde éthoxylées ;
- des résines de formaldéhyde aromatiques sulfonées ;
- des résines d'urée- ou de mélamine-formaldéhyde, par exemple des résines de mélamine-urée-formaldéhyde, des résines de mélamine-phénol-formaldéhyde ;
- des résines de polyamides, des résines de polyamines et des résines époxydes ;
(c) des copolymères AB, à titre préférentiel :
- des copolymères de styrène, par exemple des polymères de styrène-acide acrylique ou des polymères de styrène-oxyde d'éthylène ;
- des copolymères de composés de polyvinyle et d'acide maléique, par exemple un polymère de styrène-anhydride de l'acide maléique ;
- des copolymères de polyvinyle-polyalkylènes, par exemple l'acétate de vinyle ;
- un polymère d'éthylène, des polymères d'éthylène-acide acrylique-ester de l'acide acrylique ou des polymères d'éthylène-acide acrylique-acrylonitrile ;
- d'autres copolymères de vinyle, par exemple des polymères d'acétate de vinyle, des polymères d'acide acrylique-acrylonitrile, des polymères d'acide acrylique-acrylamide ;
(d) des copolymères séquencés ABA dans lesquels, à titre préférentiel :
- « A » représente des groupes solubles dans l'eau ou aptes à gonfler dans l'eau, tels que l'oxyde de polyéthylène, l'alcool polyvinylique, le polyacrylamide, l'acide polyacrylique, la polyvinylpyrolidone ou la polycaprolactone ;
- « B » représente des groupes peu ou guère solubles dans l'eau, tels que l'oxyde de polypropylène, l'acétate de polyvinyle, le polyvinylbutyral, le polyméthacrylate de lauryle, le polystyrène, l'acide polyhydroxystéarique, le polysiloxane ;
(e) des (co)polymères greffés B(A)ₙ dans lesquels, à titre préférentiel :
- « A » représente des groupes solubles dans l'eau ou aptes à gonfler dans l'eau, tels que l'alcool vinylique, des acétates de vinyle, des oxydes d'éthylène, l'oxyde de propylène, des vinylsulfonates, des acides acryliques et des vinylamines ; et
- « B » représente des chaînes de polymère vinylique ou des chaînes de siloxane ;
(f) des polymères naturels ou naturels dérivés, par exemple des dérivés de la cellulose tels que la carboxyméthylcellulose, l'hydroxypropylméthylcellulose, la méthylcellulose, l'éthylcellulose, l'acétate de cellulose, le nitrate de cellulose, des composés d'albumine (la gélatine, l'albumine, la caséine).

5. Support selon l'une quelconque des revendications 1 à 4, **caractérisé en ce que** le support recouvert de microcapsules est en outre enduit, de préférence poudré et/ou muni d'un film.

6. Support selon l'une quelconque des revendications 1 à 5, que l'on obtient par mélange d'une suspension aqueuse de microcapsules parfumées avec un support inorganique soumis à un séchage excessif dans un mélangeur à faible apport d'énergie, de préférence à un mélangeur à ailettes.

7. Support selon la revendication précédente 6, **caractérisé en ce que** le support inorganique soumis à un séchage excessif présente une teneur en eau < 10 % en poids, de préférence < 8, de manière avantageuse < 6, de manière encore plus avantageuse < 5, rapportés au support inorganique mis en oeuvre soumis à un séchage excessif.

8. Support selon l'une quelconque des revendications 6 ou 7, **caractérisé en ce que** la suspension aqueuse de microcapsules parfumées contient au moins 30 % en poids, de préférence au moins 40 % en poids, en particulier au moins 50 % en poids de microcapsules.

9. Composition de détergent, contenant :
- (A) un ou plusieurs supports selon l'une quelconque des revendications 1 à 8 ;
- (B) de 0,01 % en poids à 95 % en poids d'un ou de plusieurs agents tensioactifs supplémentaires.

10. Procédé pour l'immobilisation de microcapsules parfumées sur une matière de support inorganique, **caractérisé en ce qu'**on mélange une suspension aqueuse de microcapsules parfumées dans un mélangeur avec un support inorganique soumis à un séchage excessif.

11. Procédé selon la revendication 10, dans lequel, dans le support inorganique soumis à un séchage excessif, le pouvoir théorique maximal de fixation à l'eau du support, respectivement de la totalité de ses constituants, est dépassé vers le bas à concurrence d'au moins 10 %, de préférence à concurrence d'au moins 20 %, en particulier à concurrence d'au moins 30 %.

12. Procédé pour l'application de microcapsules parfumées qui libèrent leur contenu en cas d'exposition à une influence thermique, mécanique, chimique ou enzymatique, sur des textiles par traitement de ces textiles dans un bain de traitement des textiles, auquel on ajoute des supports particulaires selon l'une quelconque des revendications 1 à 8 ou une composition de détergent selon la revendication 9.
